# EUROPEAN PATENT APPLICATION

(11) **EP 3 545 885 A1**
(43) Date of publication of application: **02.10.2019**
(21) Application number: 19174751.8
(22) Date of filing: 31.08.2006
(51) Int. Cl.: A61B 17/70, A61F 2/44, A61F 2/30, A61B 17/00

(54) **FACET PROSTHETIC DEVICES**

(30) Priority: 23.09.2005 US 23448105
(62) Divisional of application: 16186386.5
(71) Applicant: Spinal Kinetics, Inc., Sunnyvale CA 94085 (US)
(72) Inventor: GITTINGS, Darin C., Sunnyvale, CA California 94086 (US); REO, Michael L., Redwood City, CA California 94061 (US); ROBINSON, Janine C., Half Moom Bay, CA California 94086 (US)
(74) Representative: J A Kemp

(57) **Abstract**

An inter-spinous process spacer device comprising:
upper (180) and lower (182) end plates,
at least one compressible core assembly extending between and interconnecting said upper and lower end plates, said compressible core assembly comprising a compressible core member (184) comprising one or more polymeric materials, and a plurality of fibers wound between and interconnecting said upper and lower end plates, wherein the compressible core assembly is configured to stabilize the spacing between the first spinous process and the second spinous process, to provide compressible resistance to compression, and to permit rotation, and to permit lateral bending, and
a spacer (342) comprising one or more polymeric materials and including relatively wider side sections and a relatively narrower section, containing said upper and lower end plates and at least one compressible core assembly.

## Description

### Background of the Invention

The spine is comprised of twenty-four vertebrae that are stacked one upon the other to form the spinal column. The spine provides strength and support to allow the body to stand and to provide flexibility and motion. A Section of each vertebrae includes a passageway that provides passage of the spinal cord through the spinal column. The spine thereby encases and protects the spinal cord. The spinal cord also includes thirty-one pairs of nerve roots that branch from either side of the spinal cord, extending through spaces between the vertebrae known as the neural foramen.

An intervertebral disc is located between each pair of vertebrae. The disc is composed of three component structures: (1) the nucleus pulposus; (2) the annulus fibrosus; and (3) the vertebral endplates. The disc serves several purposes, including absorbing shock, relieving friction, and handling pressure exerted between the superior and inferior vertebral bodies associated with the disc. The disc also relieves stress between the vertebral bodies, which stress would otherwise lead to degeneration or fracture of the vertebral bodies.

Disorders of the spine comprise some of the costliest and most debilitating health problems facing the populations of the United States and the rest of the world, costing billions of dollars each year. Moreover, as these populations continue to age, the incidence of spinal disorders will continue to grow. Typical disorders include those caused by disease, trauma, genetic disorders, or other causes.

The state of the art includes a number of treatment options. Medicinal treatments, exercise, and physical therapy are typical conservative treatment options. Less conservative treatment options include surgical intervention, including microdiscectomy, kyphoplasty, laminectomy, dynamic stabilization, disc arthroplasty, and spinal fusion. Traditionally, these treatment options have been applied in isolation, rather than in combination, using the most conservative treatment option that will provide a desired result.

United States Provisional Patent Application Serial No. 60/713,671, entitled "Prosthetic Intervertebral Discs," ("the '671 application"), was filed September 1, 2005, and is assigned to Spinal Kinetics, Inc., the assignee of the present application. The '671 application describes, inter alia, a treatment option that combines a prosthetic intervertebral disc with a dynamic stabilization system. The '671 application is incorporated by reference herein in its entirety.

In 1992, Panjabi introduced a model of a dynamic spinal stabilization system that describes the interaction between components providing stability in the spine. This model defined spinal instability in terms of a region of laxity around the neutral resting position of a "spinal segment, identified as the neutral zone." Panjabi, M M., "The stabilizing system of the spine. Part II. Neutral zone and instability hypothesis." J Spinal Disord 5 (4): 390-397, 1992b. There is some evidence that the neutral zone can be increased in cases of intervertebral disc degeneration, spinal injury and spinal fixation. Id. Panjabi has subsequently described dynamic stabilization systems that provide increased mechanical support while the spine is in the neutral zone and decreased support as the spine moves away from the neutral zone. See United States Published Patent Application No. 2004/0236329, published November 25, 2004, which is hereby incorporated by reference herein.

The need remains for improved spinal stabilization systems, combinations of systems, and methods for their use.

### Summary of the Invention

Spinal stabilization components, systems, and methods are provided. The spinal stabilization components are suitable for use individually, together, or with other known spinal stabilization components and systems.

In a first aspect, foramenal spacers and methods for their use are described. The foramenal spacer includes a member that has a size and shape adapted for insertion into the foramen located between a pair of adjacent vertebral bodies to prevent the pair of vertebral bodies from collapsing into one another, i.e., to maintain the interpedicular spacing between the adjacent vertebral bodies. The foramenal spacer also preferably includes a passage or other member that protects the nerve root from being compressed or otherwise physically impacted as it traverses the foramen. In a first embodiment, the foramenal spacer includes an upper C-shaped member, a lower C-shaped member, and an attachment member for attaching the upper C-shaped member to the lower C-shaped member. The upper C-shaped member is adapted to be attached to the pedicle of the superior vertebral body and to extend into the foramen defined by the pair of vertebral bodies, while the lower C-shaped member is adapted to be attached to the pedicle of the inferior vertebral body and to extend into the foramen defined by the pair of vertebral bodies. When attached together, the upper and lower C-shaped members define a passageway therethrough for allowing passage of the nerve root. The attachment member may comprise a tongue and groove mechanism, a snap-fit mechanism, or other suitable mechanism for attaching the upper and lower C-shaped member together. Alternatively, the upper C-shaped member and lower C-shaped member may each be provided with surfaces adapted to butt up against one another to form a butt-joint. In still another embodiment, the C-shaped members are mated such that they allow some travel (e.g., extension) relative to each other, such as that which may be required during flexion, extension and lateral bending, and maintain the patentcy of the passageway to allow passage of the nerve root.

In a second embodiment, the foramenal spacer includes an upper segment and a lower segment. The upper segment is adapted to be attached to the pedicle of the superior vertebral body and to extend into the foramen defined by the pair of vertebral bodies, and the lower segment is adapted to be attached to the pedicle of the inferior vertebral body and also to extend into the foramen defined by the pair of vertebral bodies. The interior surface of one of the upper segment or the lower segment and the external surface of the other of the upper segment or the lower segment define a pair of rounded, mating surfaces that together define a bearing structure that allows the upper segment to pivot relative to the lower segment. The upper segment and lower segment thereby act as a bearing to define a center of rotation. Once the upper segment and lower segment are attached to the respective vertebral bodies and are engaged with one another, the foramenal spacer provides a supporting structure that also protects the nerve root traversing the foramen, and that allows the superior and inferior vertebral bodies to pivot relative to one another.

Preferably, the foramenal spacer is formed of a rigid biocompatible material, such as stainless steel, metal alloys, or other metallic materials, or a rigid polymeric material. In alternative embodiments, the foramenal spacer is provided with an outer layer formed of a soft, conformable material (e.g., an elastomeric polymer such as polyurethane) that provides conformability with the foramen geometry and allows flexion, extension and lateral bending of the spine; In still other embodiments, the foramenal spacer includes an inner liner formed of a soft and/or low-friction material to provide an atraumatic surface for passage of the nerve root.

In a second aspect, devices, systems and methods for facet joint augmentation and replacement are provided. The devices and systems are intended to stabilize the spine and to increase the foramenal space to thereby reduce the likelihood of nerve root impingement. In a first embodiment, the stabilization and increase of foramenal space is accomplished by inserting a stabilizing member into the facet joint to restore the intra-foramenal distance. The stabilizing member comprises a structure that provides shock absorbance, cushioning, and support to the facet joint. In several embodiments, the stabilizing member comprises an encapsulated cushion. In other embodiments, the stabilizing member comprises a structure having a pair of endplates separated by a resilient core member.

In a second embodiment, some or all of the facets of each of the superior and inferior vertebral bodies are removed and replaced with a facet joint implant. In several embodiments, the facet joint implant includes an upper prosthetic facet for attachment to the superior vertebral body, and a lower prosthetic facet for attachment to the inferior vertebral body. Each prosthetic facet is attached to its respective vertebral body by screws or other similar mechanisms. Each prosthetic facet joint includes a pair of facing plates and a core member located between the pair of facing plates. The prosthetic facet is constructed and attached in a manner such that it closely mimics the functionality and performance of the natural facet joint.

In a third aspect, a lateral spinal stabilization device is provided. The lateral spinal stabilization device includes an upper attachment member and a lower attachment member for attaching to the lateral surfaces of each of the superior and inferior vertebral bodies, respectively, and a stabilizing member connected and extending between each of the upper and lower attachment members. In one embodiment, the stabilizing member comprises a damping mechanism. In other embodiments, the stabilizing member comprises a pair of endplates separated by a resilient core member.

In a fourth aspect, an anterior spinal stabilization device is provided. The anterior spinal stabilization device is adapted to be attached to the anterior surfaces of a pair of vertebral bodies and to extend between the pair of vertebral bodies to provide stabilization to the anterior portion of the vertebral unit. In a first embodiment, the anterior spinal stabilization device is in the form of a spring having a structure sufficient to carry a load after implantation and attachment to the vertebral unit. The anterior stabilization device is preferably implanted by way of a minimally invasive anterior approach, although posterior and lateral approaches are also possible.

In a fifth aspect, several dynamic stabilization devices are described. Each of the dynamic stabilization devices is intended to provide a combination of stabilizing forces to one or more spinal units to thereby assist in bearing and transferring loads. In a first embodiment, a dynamic stabilization device includes a posterior spacer member that is located generally between a pair of adjacent vertebral bodies on the posterior side of the spine. The posterior spacer is preferably formed of a generally compliant material and functions to maintain spacing between the pair of adjacent vertebral bodies while allowing relative motion between the vertebral bodies. In a preferred form, the posterior spacer is generally in the form of a short cylinder, having a central through-hole to allow passage of one or more restrictor bands, which are described more fully below. The spacer may take other shapes or forms, however, depending upon the size and shape of the spinal treatment site. The dynamic stabilization device also includes one or more restrictor bands, each of which preferably comprises a loop formed of an elastic material. The restrictor band(s) each have a size and shape adapted to be attached to the spinous processes extending from the posterior of the adjacent vertebral bodies, or to be attached by an appropriate attachment mechanism to the lamina of the adjacent bodies. Once linked to the posterior of the spine, the bands provide both stability and compliance. The performance properties of the bands are able to be varied by choice of materials, size of the bands, and by the routing of the restrictor band(s) between the adjacent vertebral bodies. For example, restrictor bands that are oriented more vertically than diagonally will provide greater resistance to flexion of the spine, whereas the more diagonal orientation will provide additional resistance to torsional movements.

In other embodiments, a dynamic stabilization device is constructed and includes materials that allow the device to be adjusted post-operatively. For example, in one embodiment, the dynamic stabilization device includes a superior attachment member for attachment to the pedicle of a superior vertebral body, an inferior attachment member for attachment to the pedicle of an inferior vertebral body, and one or more spring members extending between and interconnecting the superior and inferior attachment members. In the preferred form, the spring member is formed of a shape memory material, such as nickel titanium alloy (Nitinol). The properties of the spring member may thereby be altered post-operatively by heating elements of the device, such as by applying an electric current. As the shape memory materials may be trained by heat treatment processes prior to implantation, the properties of the spring member may be altered in a known manner by application of heat in this manner. Preferably, the electric current is applied by placing leads against the spring member under X-ray or other guidance. A given spring member may be extended or contracted to provide greater or lesser load support, or to alter any other performance characteristic of the device.

In still other embodiments, a spinal stabilization device is provided that is capable of transferring reactions from one spinal segment to an adjacent segment. In this manner, the spinal stabilization device transfers loads and reactions in the same manner as is done by the natural spinal segments operating properly. The spinal stabilization device includes at least one fixation member associated with each vertebral body, and a linkage member extending between each pair of superior and inferior fixation members. The fixation members each allow for rotation of the linkage members, thereby providing the ability for one vertebral segment to be loaded (or unloaded), either in compression or torsion, based upon the activity being undergone at an adjacent vertebral segment.

In still other embodiments, a dynamic stabilization device includes a combination of an interspinous stabilization member and one or more pedicle based stabilization members. In a preferred form, the one or more pedicle based stabilization members function by biasing the pair of adjacent vertebral bodies apart, while the interspinous stabilization member functions by biasing together the spinous processes of the adjacent pair of vertebral bodies. The combined action of the interspinous member and the pedicle based member(s) is to create a moment that relieves pressure from the disc.

In still other embodiments, a dynamic stabilization device is provided and is attached to a pair of adjacent vertebral bodies at or near one or both pairs of transverse processes extending from the pair of vertebral bodies. For example, at least one superior attachment member, such as a screw, is attached to a transverse process of the superior vertebral body, at least one inferior attachment member, such as a screw, is attached to the transverse process of the inferior vertebral body, and a loading member extends between and interconnects the superior and inferior attachment members. The attachment members may optionally extend through the transverse processes into the vertebral bodies, or they may be attached to the vertebral bodies adjacent to the transverse processes.

In still other embodiments, a dynamic stabilization device is attached such that the stabilization member is located externally of the patient's skin surface. In these embodiments, the stabilization member is attached to a pair of adjacent vertebral bodies by a pair of screws that extend through the patient's skin and into the pair of vertebral bodies. The stabilization member is then attached to and extends between the pair of screws on the exterior of the patient. The device is preferably fully adjustable.

In yet other embodiments, a dynamic stabilization device is provided and includes a fill-type adjustment mechanism. The device includes a superior attachment member that is preferably attached to the spinous process of a superior vertebral body, an inferior attachment member that is preferably attached to the spinous process of an inferior vertebral body, and a stabilization member that extends between and interconnects the superior and inferior attachment members. The attachment members may include screws, or other suitable attachment mechanisms. Interposed between at least one of the attachment members and the stabilization member is a pot. As the pot is filled, such as by injecting a biocompatible material such as bone cement containing polymethylmethacrylate (PMMA), the added volume occupied in the pot decreases the operating length of the stabilization member, thereby also changing the performance characteristics of a given stabilization member. Thus, adding material to the pot provides the ability to adjust the device post-operatively. Preferably, the post-operative adjustment may be done percutaneously.

In still other embodiments, a dynamic stabilization device includes an intervertebral spacer having an integrated stabilizing disc, the combined unit being interposed between the spinous processes of a pair of adjacent vertebral bodies.

Each of the foregoing devices, structures, and methods is adapted to be used independently, or in combinations of two or more. Preferably, several devices, structures, or methods are used in combination to obtain desired results. In particular, each of the foregoing devices may be used in combination with a prosthetic intervertebral disc to obtain desired therapeutic results.

Other and additional devices, apparatus, structures, and methods are described by reference to the drawings and detailed descriptions below.

### Brief Descriptions of the Figures

The Figures contained herein are not necessarily drawn to scale, with some components and features being exaggerated for clarity.
Figure 1 is a lateral view of a pair of adjacent vertebral bodies, including representation of the foramen and nerve roots traversing the foramen.
Figures 2A-G are illustrations of foramenal spacers in accordance with the present invention.
Figure 3 is a posterior view of a pair of adjacent vertebral bodies, including representation of the facets and facet joints.
Figure 4 is a perspective view of an embodiment of a facet joint stabilizing member.
Figure 5 is a side view of another embodiment of a facet joint stabilizing member shown implanted in a facet joint.
Figures 6A-B are illustrations of prosthetic facets.
Figure 7 is an illustration of a portion of a spinal column having a plurality of prosthetic facets in place of the native facets.
Figure 8 is a lateral view of a pair of vertebral bodies having a lateral stabilization device implanted therebetween.
Figure 9A is a lateral view of a pair of vertebral bodies having an anterior stabilization device and a posterior stabilization device implanted therebetween.
Figure 9B is an illustration of an anterior stabilization device.
Figure 10A is an illustration of a spacer member.
Figures 10B-D are illustrations of posterior dynamic stabilization devices including a spacer member and restrictor bands.
Figure 11 is a posterior view of another dynamic stabilization system.
Figure 12 is a posterior view of another dynamic stabilization system.
Figure 13 is a lateral view of another dynamic stabilization system.
Figure 14 is a posterior view of another dynamic stabilization system.
Figure 15 is a lateral view of another dynamic stabilization system.
Figure 16 is a lateral view of another dynamic stabilization system.
Figure 17 is a lateral view of another dynamic stabilization system.
Figure 18 is a three dimensional cross-sectional view of an exemplary prosthetic intervertebral disc.

### Descriptions of the Preferred Embodiments

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to at least the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "an," and "the" include plural referents unless the context clearly dictates otherwise.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present inventions.

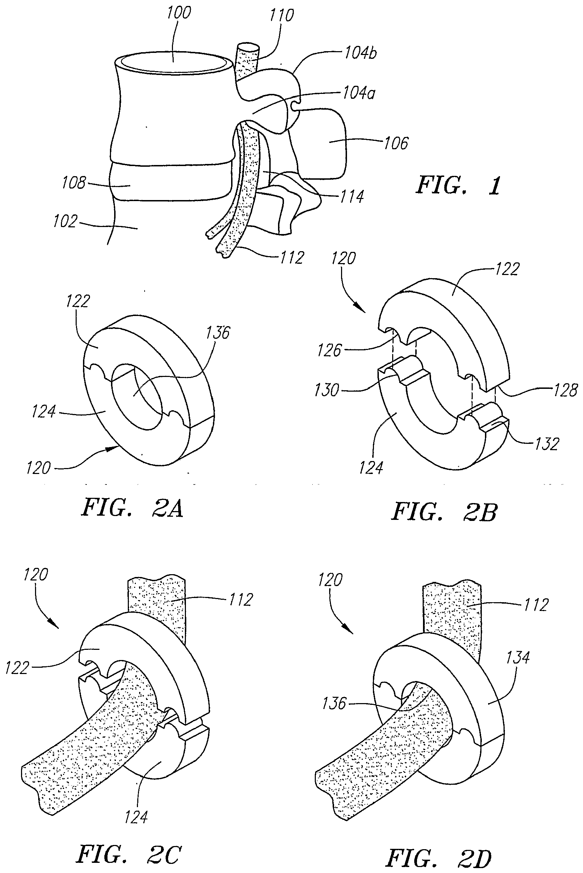

Turning now to the drawings, FIG. 1 illustrates a pair of adjacent vertebral bodies, including a superior vertebral body 100 and an inferior vertebral body 102. Each vertebral body includes a pair of transverse processes 104a-b and a spinous process 106 extending generally posteriorly from each vertebral body 100, 102. A disc 108 is located between the superior vertebral body 100 and the inferior vertebral body 102. The spinal cord 110 extends through a central passage formed by the spinal column, and nerve roots 112 transverse the foramenal space 114 defined by the pair of vertebral bodies.

When the disc is damaged due to trauma, disease, or other disorder, the superior vertebral body 100 and inferior vertebral body 102 tend to collapse upon each other, thereby decreasing the amount of space formed by the foramen 114. This result also commonly occurs when the vertebral bodies are afflicted with disease or are fractured or otherwise damaged. When the foramenal space is decreased, the vertebral bodies 100, 102 tend to impinge upon the nerve root 112, causing discomfort, pain, and possible damage to the nerve root. The foramenal spacers described herein are intended to alleviate this problem by maintaining the foramenal opening and otherwise protecting the nerve root from impingement by the vertebral bodies.

Turning to FIGS. 2A through 2G, several foramenal spacer embodiments are shown. In a first embodiment, shown in FIGS. 2A-D, the foramenal spacer 120 includes a superior C-shaped member 122 and an inferior C-shaped member 124. The pair of C-shaped members preferably include an attachment mechanism or a pair of mating surfaces. For example, as shown in FIG. 2B, the superior C-shaped member 122 is provided with a groove 126 on each of its inferior-facing surfaces 128, whereas the inferior C-shaped member 124 includes a mating tab 130 on each of its superior-facing surfaces 132. Alternatively, the tabs may be place on the superior C-shaped member and the grooves on the inferior C-shaped member, or still other attachment members may be used, such as a snap-fit mechanism or other similar structure. In still other embodiments, the mating surfaces 128, 132 may simply butt up against one another to form a butt-joint that prevents collapse of the foramenal space. When combined, the pair of C-shaped members define a generally disc-shaped member 134 having a central through-hole 136. The central through-hole 136 has a size and shape adapted to allow the nerve root 112 to pass without impingement as shown, for example, in FIGS. 2C and 2D.

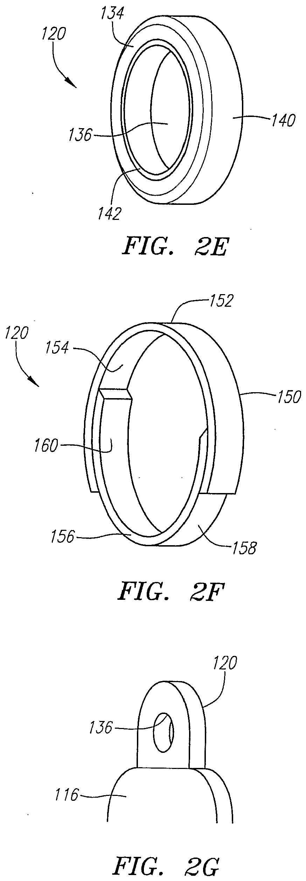

Turning to FIG. 2E, the foramenal spacer 120 may be provided with an outer layer 140 that includes a coating of a soft, conformable material. The outer layer 140 preferably covers all of the external-facing surfaces of the foramenal spacer 120, and particularly those that are positioned to engage the vertebral body surfaces. The outer layer 140 is preferably formed of a soft, conformable biocompatible material such as silicone, polyurethane, or other similar polymeric materials, and may be applied to the foramenal spacer 120 by methods well-known in the art. The outer layer 140 may provide structural protection to the vertebral bodies forming the foramenal space, and also allows the foramenal spacer 120 to adapt to the varying foramenal geometries formed by the vertebral bodies.

An optional inner layer or liner 142 may be provided on the exposed surfaces defining the through-hole 136. The inner layer or liner 142 is preferably formed of a coating of soft and/or low-friction material to provide an atraumatic surface for passage of the nerve root 112. Preferably, the inner layer or liner 142 is formed of similar materials as those used for the outer layer 140, including silicone, polyurethane, or other polymeric materials. Alternatively, the inner layer or liner 142 may comprise a coating of polyethylene, PTFE, or other similar material.

In addition, an optional spring member, gasket, cushion, or other similar material or device (not shown in the drawings) may be interposed between the superior C-shaped member 122 and the inferior C-shaped member 124. Preferably, the spring member (or the like) may be located on the abutting surfaces of the two C-shaped members. This spring member (or the like) provides the spacer 120 with the capability of vertical expansion and contraction as the spring member extends and compresses, thereby providing a range of motion for supporting the foramenal space.

Turning now to FIG. 2F, another foramenal spacer embodiment is shown. In this embodiment, the foramenal spacer 120 includes an upper segment 150 and a lower segment 156. The upper segment 150 includes an external surface 152 that has a shape adapted to engage the portion of the superior vertebral body defining the foramenal space 114. Similarly, the lower segment 156 includes an external surface 158 that has a shape adapted to engage the portion of the inferior vertebral body defining the foramenal space 114. An internal surface 154 of the upper segment 150 includes a curved portion that is adapted to rotatably engage a mating curved portion of the external surface 158 of the lower segment 156. In this way, the upper segment 150 and lower segment 156 are rotationally connected to one another, i.e., the upper segment 150 and lower segment 152 function similar to a bearing having a center of rotation. When the upper segment 150 is attached to the superior vertebral body 100, and the lower segment is connected to the inferior vertebral body 102, the foramenal spacer 120 allows the two vertebral bodies to pivot relative to one another, thereby providing an additional range of motion. The foramenal spacer 120 shown in FIG. 2F also optionally includes the outer layer 140 and inner layer or liner 142 described above in relation to FIG. 2E.

The foramenal spacer 120 may be implanted by any appropriate surgical technique, including accessing the foramenal space by either a posterior approach or a lateral approach. The lateral approach is believed to provide optimal access to find exposure of the foramen, but techniques for posterior lumbar interbody fusion (PLIF) and transforamenal lumbar interbody fusion (TLIF) also provide sufficient access. Once access is gained, the foramenal spacer 120 is preferably attached to the pedicle or other anatomic structure that allows extension of the spacer into the foramenal space 114. For example, the foramenal spacer 120 may be press fit into the foramen 116, as illustrated in FIG. 2G, or a tab (not shown) may be provided for attaching the foramenal spacer 120 to the pedicle or other anatomical structure.

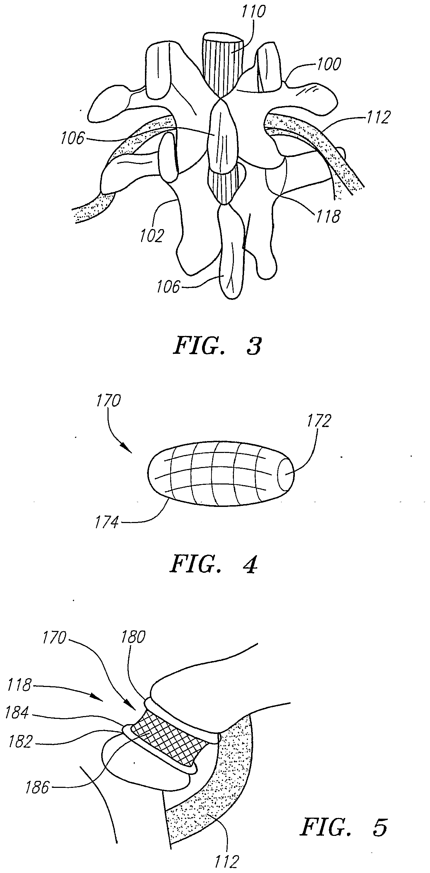

Turning next to FIG. 3, a posterior view of a pair of adjacent vertebral bodies is shown. The Figures illustrates a superior vertebral body 100 and an inferior vertebral body 102. Each vertebral body includes a pair of transverse processes 104a-b and a spinous process 106 extending generally posteriorly from each vertebral body 100, 102. The spinal cord 110 extends through a central passage formed by the spinal column, and nerve roots 112 transverse the foramenal space 114 defined by the pair of vertebral bodies. A facet joint 118 is formed by a pair of facing facets, one each from the superior and inferior vertebral bodies.

Several of the known devices and systems for posterior spinal stabilization are designed and provide the function of opening the foramen or maintaining the foramenal spacing in order to off-load the nerve that traverses the foramen. This is commonly done by attaching a device to the pedicles of each of the vertebral bodies and providing a distracting force between the attachment members. Several alternative and novel devices and methods are described herein.

Turning to FIG. 4, a facet stabilizing member 170 is shown. The facet stabilizing member 170 preferably includes a core member 172 encased in a jacket 174. The core member 172 is preferably formed of a hydrogel, polyurethane, or other polymeric material suitable for providing the shock absorbing and spacing function necessary to stabilize the facet joint. The jacket 174 may be a woven fabric of biocompatible material and is intended to maintain the integrity and shape of the core member 172 and to otherwise provide structural strength to the facet stabilizing member 170. The facet stabilizing member 170 has a size and shape adapted to be placed in the facet joint 118 to thereby provide stabilization to the joint and to prevent collapse of the foramenal space.

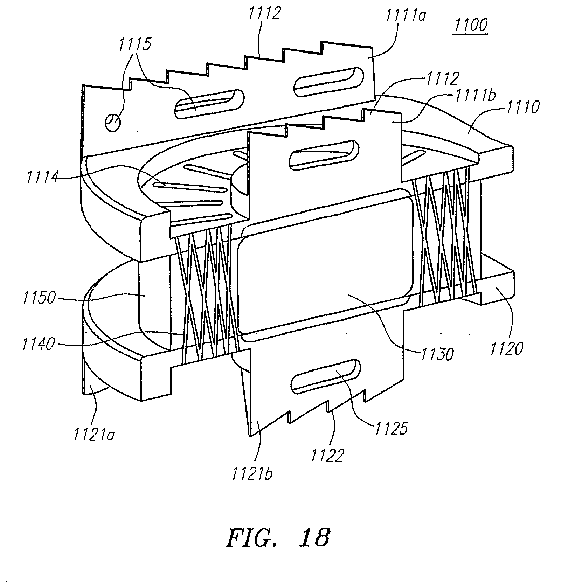

Turning to FIG. 5, another embodiment of a facet stabilizing member 170 is shown. In this embodiment, the spinal stabilizing member 170 includes an upper endplate 180, a lower endplate 182, and a core member 184 extending between and interconnecting the upper endplate 180 and lower endplate 182. Preferably, the facet stabilizing member also includes a plurality of fibers 186 wound between and interconnecting the upper endplate 180 and lower endplate 182. The construction and materials of the facet stabilizing member 170 shown in FIG. 5 are similar to the construction and materials of the prosthetic intervertebral disc described below in relation to FIG. 18, and to several of the prosthetic intervertebral discs described in United States Patent Application Serial No. 10/903,276, filed July 30, 2004, and United States Patent Application Serial No. 60/713,671, filed September 1, 2005, each of which applications is hereby incorporated by reference herein. Other prosthetic discs described in the foregoing applications may also be adapted for use as a facet stabilizing member 170 as described herein. The size of the facet stabilizing member 170 is typically smaller than the sizes of the prosthetic discs described in the foregoing applications, but the overall construction of the structure is preferably the same.

The facet stabilizing member 170 is implanted between the pair of opposed facets associated with the pair of adjacent vertebral bodies. Additional features, such as fins, fixation members, or other structures (not shown), may also be incorporated on the facet stabilizing member 170 to limit translation. The facet joint is synovial, therefore requiring implantation through the capsule. Access to the facet joint is obtained by any of the methods described above in relation to implantation of the foramenal spacer.

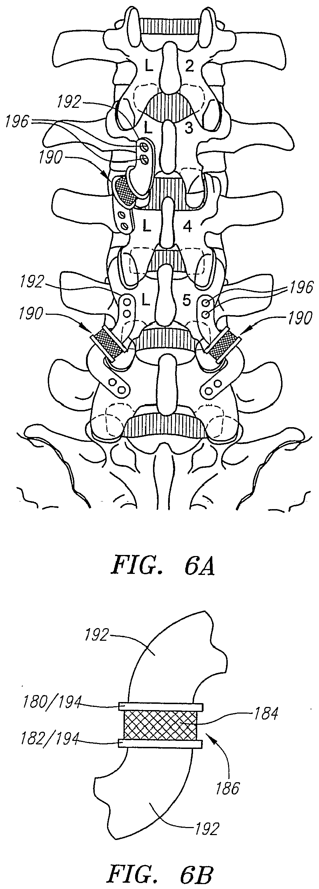

Turning to FIGS. 6A-B, prosthetic facets and facet joints are shown. During many spinal surgical procedures, particularly those including approach by the posterior, some or all of the facet is removed to provide access to implant one or more prosthetic structures. Similarly, many spinal procedures create loss of height of the disc or similar unintended results. In these cases, or in cases in which the facets or facet joints become damaged through trauma, disease, or other disorder, it may be desirable to replace some or all of the facet with a prosthetic device to restore stabilization to the affected spinal segments.

In FIG. 6A, a number of prosthetic facets 190 are shown as implanted in several locations on a spinal column. Each prosthetic facet 190 includes an attachment arm 192 that is generally elongated and curved to match the shape and structure of the native facet. The attachment arm 192 terminates in an endplate 194 that mimics the facing surface of the native facet. The attachment arm 192 is attached to its associated vertebral body by one or more screws 196 or other suitable attachment mechanism. A facet stabilizing member 170, identical to those described above in relation to FIG. 5, is interposed between a pair of prosthetic facets 190, with the facet endplates 194 serving as the endplates for the facet stabilizing member 170. (See, in particular, FIG. 6B). As shown in FIG. 6A, the prosthetic facet joint is oriented to be on plane with the native facet. Thus, the orientation of the facet joint will vary between vertebral segments.

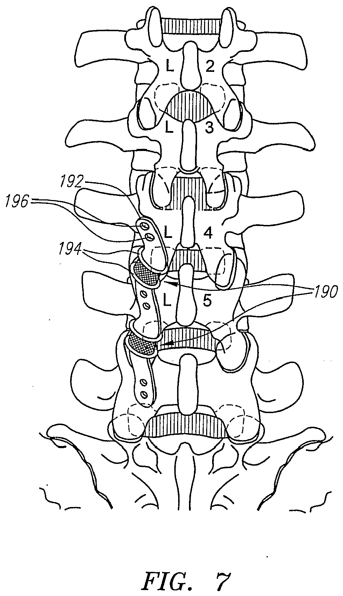

FIG. 7 illustrates a multi-level stabilization over several adjacent vertebral segments using the prosthetic facets 190. A first prosthetic facet 190 is attached to the sacrum 119, and additional prosthetic facets 190 are attached to the L5 and L4 vertebrae.

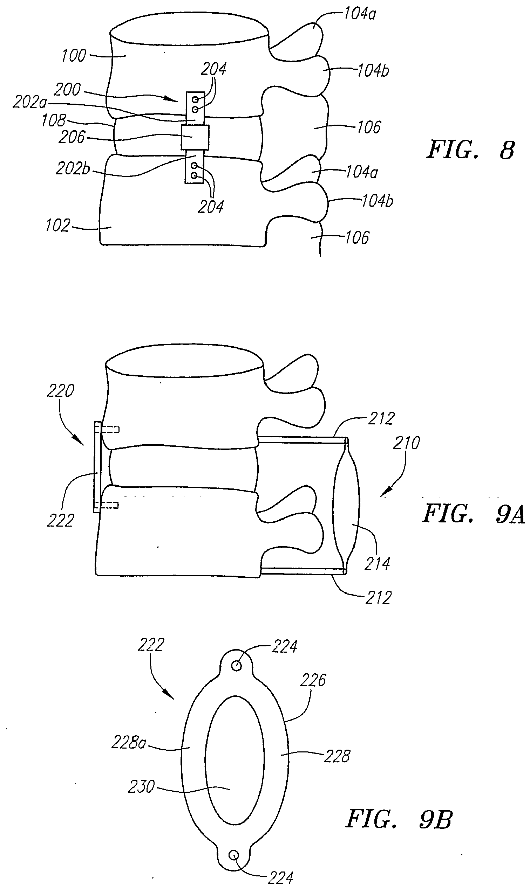

Turning next to FIG. 8, a lateral stabilization device is shown. The lateral stabilization device 200 includes an upper attachment arm 202a adapted to be attached to the superior vertebral body 100 by one or more screws 204 or other attachment mechanism, and a lower attachment arm 202b adapted to be attached to the inferior vertebral body 102 by one or more screws 204 or other attachment mechanism. The device also includes a stabilization member 206. The stabilization member 206 may include a spring, a combination of springs, a damping mechanism, or other mechanism that provides a desired stabilization function. In a preferred embodiment, the stabilization member includes a structure identical to the facet stabilization member 170 described above in relation to FIG. 5.

Advantageously, the lateral stabilization device 200 is attached to the lateral surfaces of the pair of adjacent vertebral bodies 100, 102. In a particularly preferred embodiment, a lateral stabilization device 200 is attached on both lateral sides of the pair of vertebral bodies.

FIG. 9 shows a pair of adjacent vertebral bodies 100, 102 having both a posterior stabilization device 210 and an anterior stabilization device 220 attached to each of the pair of vertebral bodies. The posterior stabilization device 210 includes a pair of pedicle screws 212, one attached to each of the superior vertebral body 100 and the inferior vertebral body 102. A stabilization member 212 extends between and interconnects the pair of pedicle screws 212. The stabilization member 214 may comprise a load bearing dynamic structure that is spring loaded, that includes a damping member, or any combination of such structures. The anterior stabilization device 220 includes an anterior element 222, the details of which are best shown in FIG. 9B. The anterior element 222 is preferably formed of a material having superelastic properties, and includes a shape that allows the anterior element 222 to be constrained for a minimally invasive implantation procedure. As shown, the anterior element 222 includes an attachment hole 224 at each end, and a central portion 226 that includes a pair of side bands 228a-b that define a central aperture 230. The anterior element 222 may be rolled or compressed into a low profile contracted state for implantation. Once introduced, the anterior element is partially released from the contracted state and attached to the pair of vertebral bodies adjacent to the damaged disc 108. The anterior element 222 is preferably attached by screws or other suitable mechanisms. Once attached, the anterior element 222 is fully extended to its operative state and is capable of bearing loads to provide stabilization to the vertebral segments.

The anterior stabilization device 220 may be used alone, in combination with the posterior stabilization device 210 illustrated in FIG. 9A, or in combination with any other suitable stabilization device or structure. By using a combination of stabilization devices, it may be possible to provide additional amounts or types of stabilization and unloading of the vertebral segment than is possible by use of only a single stabilization structure.

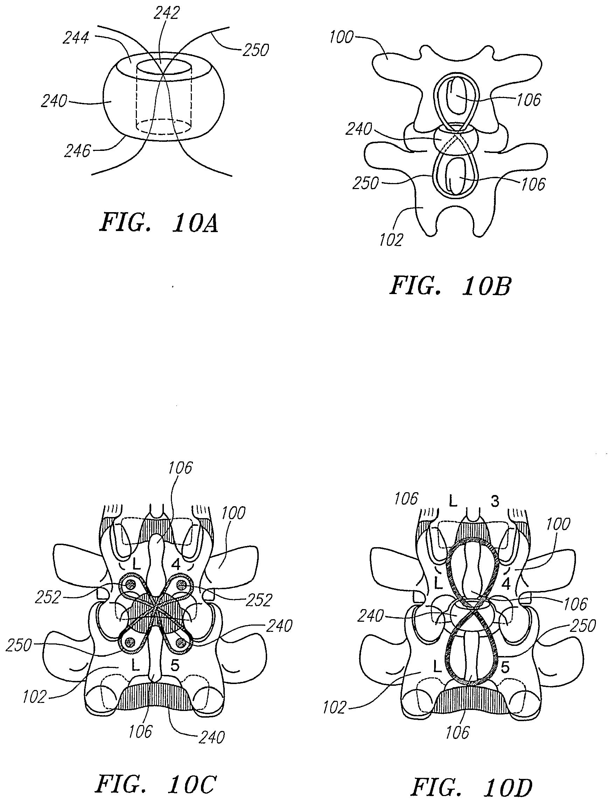

Turning now to FIGS. 10A-D, several embodiments of a posterior dynamic stabilization device are shown. The dynamic stabilization devices include a posterior spacer and one or more restrictor bands. As explained below, the spacer may be integrated with the restrictor band(s), or it may be provided independently of the restrictor band(s).

Turning first to FIG. 10A, a posterior spacer 240 is shown. The posterior spacer 240 is generally in the shape of a short cylinder, having a central through-hole 242 and an upper surface 244 and lower surface 246. The posterior spacer 240 may optionally be provided in any other form or shape, as described more fully below. The spacer 240 is preferably formed of a generally compliant biocompatible material, such as a polyurethane, silicone, or other suitable polymeric material. As shown in FIGS. 10B-D, the spacer 240 is generally located between the spinous processes of a pair of adjacent vertebral bodies. The spacer maintains the spacing between the vertebral bodies while allowing a desired amount of relative motion between the two vertebral bodies.

The restrictor band(s) 250 are each preferably formed in a continuous loop and are formed of a relatively elastic biocompatible material, such as any number of elastomeric and/or polymeric materials suitable for the purpose. The restrictor band(s) 250 are linked to the posterior spine to provide both stability and compliance. The band(s) 250 are attached either to the lamina by way of attachment screws 252 or other suitable attachment mechanisms (see FIG. 10C, or they are looped directly onto the spinous processes 106 of the pair of vertebral bodies (see FIGS. 10B, 10D). The materials, sizes, structures, and routing of the restrictor band(s) 250 are able to be tailored to obtain a desired type and degree of constraint. For example, a routing pattern that is oriented relatively more diagonally, as in FIG. 10C, will provide more resistance to torsional movement than will a routing pattern that is oriented more vertically, as in FIG. 10D. Other routing variations are also possible, as will be recognized by those skilled in the art.

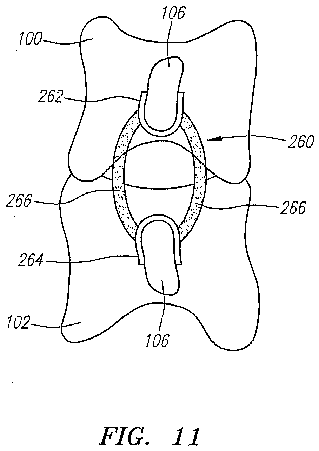

Turning now to FIG. 11, another embodiment of a dynamic spinal stabilization device is shown. The device includes a construction and that provides the capability of adjusting the performance characteristics of the stabilization device after it has been implanted. In the illustrated embodiment, the spinal stabilization device 260 includes a superior attachment member 262 and an inferior attachment member 264, for attachment to a superior vertebral body 100 and an inferior vertebral body 100, respectively. The superior attachment member 262 and inferior attachment member 264 may be attached to the spinous processes 106 of the respective vertebral bodies 100, 102, as shown, or they may be attached to the pedicles or other suitable portions of the vertebral bodies. The attachment members 262, 264 preferably comprise screws, although other attachment members may be used as desired or as suitable. The stabilization device 260 includes one or more spring elements 266 that each extend between and interconnect the superior attachment member 262 and inferior attachment member 264. Each spring element 266 is preferably formed of nickel titanium alloy (Nitinol) or other suitable biocompatible shape memory material. The shape and properties of each spring element 266 are able to be altered at any time, either prior to implantation or after implantation, by heating the spring element, for example, using an electric current. The shape memory material may be trained by a heating process to conform to a predetermined shape upon being heated to a predetermined temperature, in a manner known to those skilled in the art. Thus, the user is able to alter the shape, size, or performance characteristics of the spring elements 266 through application of heat to those members. For example, leads may be placed in contact with the spring elements 266 under X-ray or other guidance after implantation in the spine of a patient. Electric current is then supplied to the spring elements 266 through the leads, allowing the user to alter the size, shape, or performance characteristics of the spring elements.

Although the spring elements 266 shown in the embodiment illustrated in FIG. 11 are generally straight struts, the spring elements 266 may alternatively be provided in any shape, size, or orientation suitable for a given application.

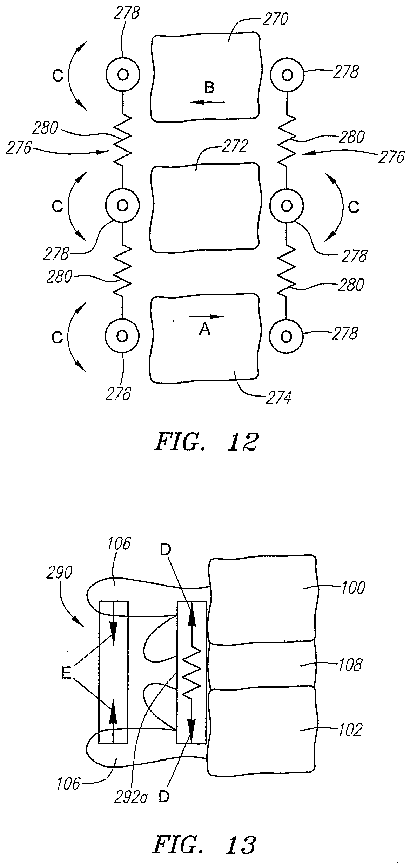

Turning next to FIG. 12, another spinal stabilization device is shown in a generally schematic representation. The spinal stabilization device illustrated in FIG. 12 is adapted to transfer loads from one motion segment to adjacent segments. Three adjacent vertebral bodies 270, 272, and 274 are shown schematically in the Figure. A pair of interconnected stabilization devices 276 are attached to each of the three vertebral bodies. Each stabilization device 276 includes a fixation element 278 attached to each vertebral body and a linkage 280 extending between and attached to each adjacent pair of fixation elements 278.

Each of the fixation elements 278 comprises a bearing structure or similar mechanism that provides the capability of rotating the attached linkage 280. This allows a first vertebral segment, such as vertebral body 270, to be loaded in reaction to a load placed upon the adjacent vertebral segments, such as vertebral bodies 272 and 274. As a non-limiting example, when the lowest vertebral body 274 moves to the right, as shown by arrow "A", the transfer of this load through rotation of the fixation elements 278 imposing loading upon the attached linkages 280 influences the upper vertebral body 270 to move to the left, as shown by arrow "B". This movement is consistent with the natural movement of the spine when the body twists. Compression and flexion loads are transferred in a similar manner.

As noted above, each of the fixation elements 278 is preferably in the form of a bearing or similar rotatable structure that provides rotational movement as represented by the arrows "C". The linkages 280 may comprise a spring element or multiple spring elements having a size, shape, spring constant, and other characteristics that provide the desired amount of load transfer in response to rotation of the fixation elements 278. In addition, although two stabilization devices 276 are shown in the Figure, more or fewer devices may be used depending upon the degree of stabilization needed or desired. The stabilization devices 276 may also extend between more (e.g., four or more) or fewer (e.g., two) adjacent vertebral segments.

Turning next to FIG. 13, a multi-component dynamic stabilization system is shown schematically. Current dynamic spinal stabilization systems are typically either interspinous devices (i.e., connected between the spinous processes of adjacent vertebral bodies) or pedicle screw based devices (i.e., connected between pedicle screws attached to the pedicles of adjacent vertebral bodies). Each of these types of dynamic stabilization devices functions by providing a distracting force that unloads the disc 108. The system shown in FIG. 13 includes an interspinous stabilization system 290 connected to the spinous processes 106 of a pair of adjacent vertebral bodies 100, 102, and a pair of pedicle based stabilization systems 292a-b (only one of the pedicle based systems is shown in the Figure) attached by pedicle screws to the pair of adjacent vertebral bodies 100, 102 on each side of the spinous processes. Each of the pedicle based systems 292a-b comprises a spring loaded or other suitable structure that provides a distracting force, represented by arrows "D", that tends to unload the disc 108. The interspinous system 290, on the other hand, includes a spring loaded or other suitable structure that biases the spinous processes 106 of the adjacent vertebral bodies 100, 102 toward one another, as represented by arrows "E". The combined action of the interspinous system 290 and the pedicle based systems 292a-b creates a moment that relives pressure from the disc 108.

Advantageously, the interspinous system 290 and/or the pedicle based systems 292a-b of the foregoing embodiment may be constructed such that one or more of the systems is externally adjustable, as described, for example, in relation the devices illustrated in FIG. 11.

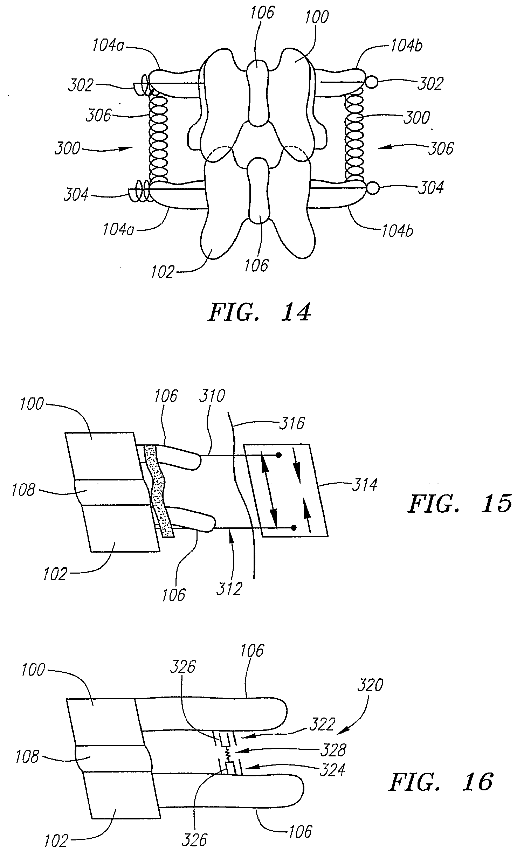

Turning next to FIG. 14, a pair of adjacent vertebral bodies 100, 102 are shown. A dynamic stabilization device 300 is attached to each of the vertebral bodies at a position at or near the transverse processes 104a-b of each vertebral body 100, 102. Each dynamic stabilization device 300 includes an upper attachment screw 302 and a lower attachment screw 304. The screws 302, 304 may be placed through the transverse process to the vertebral body, or they may be attached direction to the vertebral body adjacent the transverse process 104a-b. A loading member 306 is attached to each of the upper attachment screw 302 and the lower attachment screw 304 and extends therebetween. The loading member 306 is adapted to stabilize the adjacent vertebral bodies by providing an appropriate level of distraction or attraction forces. The loading member 306 may comprises a spring, a set of springs, a damping member, or any other suitable structure such as those described elsewhere herein.

FIG. 15 provides a schematic illustration of an externally adjustable dynamic stabilization system. The system includes an upper screw 310 and lower screw 312 extending posteriorly from a superior vertebral body 100 and an inferior vertebral body 102, respectively. Each screw extends outside of the patient's body. A stabilization member 314 is attached to each of the screws 310, 312 on the external surface of the patient's back, i.e., outside the surface of the skin 316. The stabilization member 316 may be a spring loaded, multiple spring loaded, damping mechanism, or any other suitable stabilization system such as those described elsewhere herein. Advantageously, the stabilization system 316 is readily adjustable post-operatively because it is located externally of the patient. Accordingly, any adjustments to the performance of the system may be made easily and without the need for additional surgical intervention.

FIG. 16 is a schematic illustration of another adjustable stabilization system. The spinal stabilization system 320 includes an upper pot 322 attached to the spinous process 106 of a superior vertebral body 100, and a lower pot 324 attached to the spinous process 106 of an inferior vertebral body 102. Each of the upper pot 322 and lower pot 324 forms a portion of the attachment mechanism for the stabilization device. The upper pot 322 and lower pot 324 may be attached to the spinous processes 106 by any suitable mechanism, such as one or more screws. Each of the upper pot 322 and lower pot 324 includes a cylindrical portion that is adapted to receive a connector 326 located at each of the upper end and lower end of a spring 328. Each of the connectors 326 engages one of the upper pot 322 and lower pot 324, thereby allowing the spring 328 to provide a distracting force to the vertebral bodies 100, 102.

Because the upper pot 322 and lower pot 324 are generally hollow, it is possible to partially fill one or both of the pots 322, 324 to decrease the effective length of the spring 328 extending between the pots, i.e., partially filling the pots causes the connectors to engage the filler material at a level removed from the bottom of the pot 322, 324. Either or both of the pots 322, 324 may be partially filled with bone cement containing polymethylmethacrylate (PMMA) or another suitable material. Preferably, the filling operation may be performed post-operatively by way of a percutaneous access, thereby eliminating the need for additional surgical intervention.

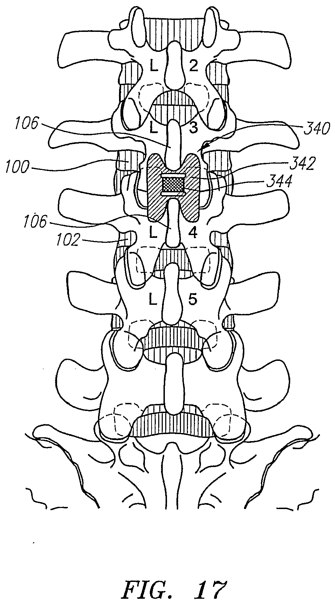

FIG. 17 provides an illustration of another dynamic stabilization system. The stabilization system 340 includes a DIAM™ type intervertebral spacer 342 interposed between the spinous processes 106 of a pair of adjacent vertebral bodies 100, 102. DIAM™ type intervertebral spacers are commercially available and are produced by Medtronic Sofamor Danek. The spacer 342 is generally "H" shaped, including a relatively narrow center section located between relatively wider side sections. This shape allows the spacer to be effectively sandwiched between the spinous processes 106 of a pair of adjacent vertebral bodies 100, 102, as shown in FIG. 17. The spacer 342 is a silicone device covered with polyethylene, and functions by reducing loading of the disc, restoring the posterior tension band, realigning the facets, and restoring the foramenal height.

In addition, a stabilizing disc 344 is interposed between the spinous processes 106 in place of a portion of the spacer 340. The stabilizing disc 344 has a structure and is constructed in a manner identical to the facet stabilizing member 170 described above in relation to FIG. 5, having a core member located between a pair of endplates. The stabilizing disc 344 allows for compression and rotation, if needed. The stabilizing disc 344 also facilitates lateral bending.

As noted above, this application incorporates by reference United States Provisional Patent Application Serial No. 60/713,671, entitled "Prosthetic Intervertebral Discs," ("the '671 application"), which was filed September 1, 2005, and which is assigned to Spinal Kinetics, Inc., the assignee of the present application. The '671 application describes, inter alia, spinal treatment methods that combine a prosthetic intervertebral disc with a dynamic stabilization system. Each of the dynamic stabilization systems described in the present application are suitable for use in combination with prosthetic intervertebral discs such as those described in the '671 application, and others described in United States Patent Application Serial No. 10/903,276, filed July 30, 2004, ("the '276 application"), which is also incorporated by reference herein.

For example, an exemplary prosthetic intervertebral disc 1100 is shown in FIG. 18, which is reproduced from FIG. 3 of the '671 application and which was also described in the '276 application. This prosthetic disc is described for exemplary purposes, and is not intended to represent the only type of prosthetic disc that is suitable for use in combination with the devices and systems described elsewhere herein. Turning to the Figure, the prosthetic disc 1100 has an integrated structure that includes an upper endplate 1110, a lower endplate 1120, and a core member 1130 retained between the upper endplate 1110 and the lower endplate 1120. One or more fibers 1140 are wound around the upper and lower endplates to attach the endplates to one another. The wind of the fibers 1140 allows a degree of axial rotation, bending, flexion, and extension by and between the endplates. An annular capsule 1150 is optionally provided in the space between the upper and lower endplates, surrounding the core member 1130 and the fibers 1140. The upper endplate 1110 and lower endplate 1120 are generally flat, planar members, and are fabricated from a biocompatible material that provides substantial rigidity.

The upper surface of the upper endplate 1110 and the lower surface of the lower endplate 1120 are preferably each provided with a mechanism for securing the endplate to the respective opposed surfaces of the upper and lower vertebral bodies between which the prosthetic disc is to be installed. For example, in Figure 18, the upper endplate 1110 includes a plurality of anchoring fins 1111a-b. The anchoring fins 1111a-b are intended to engage mating grooves that are formed on the surfaces of the upper and lower vertebral bodies to thereby secure the endplate to its respective vertebral body. The anchoring fins 1111a-b extend generally perpendicularly from the generally planar external surface of the upper endplate 1110, i.e., upward from the upper side of the endplate as shown in Figure 18. Each of the anchoring fins 1111a-b has a plurality of serrations 1112 located on the top edge of the anchoring fin. The serrations 1112 are intended to enhance the ability of the anchoring fin to engage the vertebral body and to thereby secure the upper endplate 1110 to the spine.

Similarly, the lower surface of the lower endplate 1120 includes a plurality of anchoring fins 1121a-b. The anchoring fins 1121a-b on the lower surface of the lower endplate 1120 are identical in structure and function to the anchoring fins 1111a-b on the upper surface of the upper endplate 1110, with the exception of their location on the prosthetic disc.

The anchoring fins 1111, 1121 may optionally be provided with one or more holes or slots 1115, 1125. The holes or slots help to promote bony ingrowth that assist in anchoring the prosthetic disc 1100 to the vertebral bodies.

The upper endplate 1110 contains a plurality of slots 1114 through which the fibers 1140 may be passed through or wound, as shown. The actual number of slots 1114 contained on the endplate is variable. The purpose of the fibers 1140 is to hold the upper endplate 1110 and lower endplate 1120 together and to limit the range-of-motion to mimic the range-of-motion and torsional and flexural resistance of a natural disc.

The core member 1130 is intended to provide support to and to maintain the relative spacing between the upper endplate 1110 and lower endplate 1120. The core member 1130 is made of a relatively compliant material, for example, polyurethane or silicone, and is typically fabricated by injection molding. A preferred construction for the core member includes a nucleus formed of a hydrogel and an elastomer reinforced fiber annulus. The shape of the core member 1130 is typically generally cylindrical or bean-shaped, although the shape (as well as the materials making up the core member and the core member size) may be varied to obtain desired physical or performance properties. For example, the core member 1130 shape, size, and materials will directly affect the degree of flexion, extension, lateral bending, and axial rotation of the prosthetic disc.

The annular capsule 1150 is preferably made of polyurethane or silicone and may be fabricated by injection molding, two-part component mixing, or dipping the endplate-core-fiber assembly into a polymer solution. A function of the annular capsule is to act as a barrier that keeps the disc materials (e.g., fiber strands) within the body of the disc, and that keeps natural in-growth outside the disc.

The foregoing prosthetic disc 1100, or other suitable prosthetic discs, may be implanted by surgical techniques described in the '671 and '276 applications and elsewhere. As described above, it will often be advantageous to combine the prosthetic intervertebral disc with any of the other devices, systems, and methods described herein to obtain synergistic therapeutic results in treatment of spinal disease, trauma, or other disorder.

Accordingly, it is to be understood that the inventions that are the subject of this patent application are not limited to the particular embodiments described, as such may, of course, vary. In particular, it is specifically contemplated that two or more of the specific embodiments described herein may be combined, to the extent that the embodiments are compatible with one another. Such combinations provide performance benefits that exceed those available to known devices or devices utilized independently.

Unless defined otherwise, all technnical and scientific terms used herein have the same meaning as commonly understood by one of ordinary skill in the art to which these inventions belong. Although any methods and materials similar or equivalent to those described herein can also be used in the practice or testing of the present inventions, the preferred methods and materials are herein described.

All patents, patent applications, and other publications mentioned herein are hereby incorporated herein by reference in their entireties. The patents, applications, and publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present inventions.

The preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims.

### PREFERRED EMBODIMENTS

The 17 claims of the grandparent application are reproduced immediately below as clauses. These clauses define preferred embodiments. The applicant reserves the right to pursue protection for the combinations of features set out in these clauses, and/or for any other subject-matter contained in the grandparent application as filed, either in the present divisional application or in a further application divided from the present divisional application. The claims of the grandparent application are not the claims of this divisional application. The 2 claims of the current divisional application are contained in a subsequent section headed "CLAIMS".
1. A foramenal spacer, comprising:
   a first support member having an external surface configured to engage the pedicle surface of a first vertebral body of a patient;
   a second support member having an external surface configured to engage the pedicle surface of a second vertebral body of a patient, said second vertebral body being adjacent to said first vertebral body;
   said first support member and said second member being connected together such that a foramenal height between said first vertebral body and said second vertebral body is maintained at no less than a minimum foramenal height when said foramenal spacer is interposed between said first vertebral body and second vertebral body.
2. The foramenal spacer of clause 1, wherein said first support member and said second support member define a passage therebetween.
3. The foramenal spacer of clause 2, wherein said first support member and said second support member are capable of rotational movement relative to one another.
4. The foramenal spacer of clause 2, wherein said first support member and said second support member are capable of expansive movement relative to one another.
5. A method for augmenting a facet joint between a superior vertebral body and an inferior vertebral body of a patient, comprising:
   providing a facet stabilizer, said facet stabilizer including a first endplate, a second endplate, and a core member interposed between said first endplate and said second endplate; and
   implanting said facet stabilizer between the facets comprising said facet joint.
6. A method for mammalian spinal stabilization, comprising:
   implanting a prosthetic intervertebral disc between a superior vertebral body and an inferior vertebral body of a patient; and
   implanting a stabilizer system configured to engage said superior vertebral body and said inferior vertebral body of said patient.
7. The method of clause 6, wherein said stabilizer system comprises a spacer interposed between the spinous processes of each of said superior vertebral body and said inferior vertebral body.
8. The method of clause 7, wherein said stabilizer system further comprises a restrictor band attached to each of said superior vertebral body and said inferior vertebral body.
9. The method of clause 7, wherein said stabilizer system further comprises a disc member embedded in said spacer, said disc member comprising an upper endplate, a lower endplate, and a core member located between said upper endplate and said lower endplate.
10. The method of clause 6, wherein said stabilizer system comprises a first attachment member connected to said superior vertebral body, a second attachment member connected to said inferior vertebral body, and a stabilizer member connected to each of said first attachment member and said second attachment member.
11. The method of clause 10, wherein said stabilizer member comprises a shape memory material.
12. The method of clause 10, wherein at least one of said first attachment member and said second attachment member is rotatable, and wherein rotation of at least one of said first attachment member and said second attachment member causes force from movement of one of said superior vertebral body or said inferior vertebral body to be transferred to said other of said superior vertebral body or said inferior vertebral body.
13. The method of clause 6, wherein said stabilizer system comprises a first member attached to and configured to impart a distracting force between each of said superior vertebral body and said inferior vertebral body, and a second member attached to and configured to impart an attracting force between each of said superior vertebral body and said inferior vertebral body.
14. The method of clause 10, wherein said first attachment member is connected to a transverse process of said superior vertebral body, and said second attachment member is connected to a transverse process of said inferior vertebral body.
15. The method of clause 10, wherein said stabilizer system further comprises a pot configured to receive and retain a filler material.
16. The method of clause 6, wherein said stabilizer system is attached to lateral surfaces of said superior vertebral body and said inferior vertebral body.
17. The method of clause 6, wherein said stabilizer system is attached to anterior surfaces of said superior vertebral body and said inferior vertebral body.

## Claims

1. In a system for stabilizing a human spine, the human spine having an upper vertebral body having an upper spinous process, facet joints, and foramenal spaces and a lower vertebral body having a lower spinous process, facet joints, and foramenal spaces, the upper and lower vertebral bodies being adjacent each other, an inter-spinous process spacer device comprising:
upper and lower end plates,
at least one compressible core assembly extending between and interconnecting said upper and lower end plates, said compressible core assembly comprising a compressible core member comprising one or more polymeric materials, and a plurality of fibers wound between and interconnecting said upper and lower end plates, wherein the compressible core assembly is configured to stabilize the spacing between the first spinous process and the second spinous process, to provide compressible resistance to compression, and to permit rotation, and to permit lateral bending, and
a spacer comprising one or more polymeric materials and including relatively wider side sections and a relatively narrower section, containing said upper and lower end plates and at least one compressible core assembly.

2. The device of claim 1 wherein the spacer is configured by sizing such that, when implanted between the first spinous process and the second spinous process, the spacer realigns the facets associated with the upper vertebral body and the lower vertebral body and restores the foramenal height associated with the upper vertebral body and the lower vertebral body.
